# EUROPEAN PATENT SPECIFICATION

(11) **EP 3 230 434 B1**
(45) Date of publication and mention of the grant of the patent: **22.05.2019**
(21) Application number: 15839147.4
(22) Date of filing: 14.12.2015
(51) Int. Cl.: C12M 1/107, C12M 1/00, C12M 1/02

(54) **METHOD FOR ANAEROBIC DIGESTION AND DEVICE FOR IMPLEMENTING SAID METHOD**
VERFAHREN ZUR ANAEROBER FAULUNG UND VORRICHTUNG ZUR IMPLEMENTIERUNG DIESES VERFAHRENS
PROCÉDÉ DE DIGESTION ANAÉROBIE ET DISPOSITIF POUR LA MISE EN OUVRE DUDIT PROCÉDÉ

(30) Priority: 12.12.2014 IT MI20142125
(43) Date of publication of application: 18.10.2017
(73) Proprietor: LAVANGA, Vito, I-20017 Rho (MI) (IT)
(72) Inventor: FARNE', Stefano, 20128 Milano (IT)
(74) Representative: Garavelli, Paolo
(86) International application number: PCT/IT2015/000306
(87) International publication number: WO 2016/092582

(56) References cited:
- WO-A1-2010/102746
- WO-A1-2014/075192
- US-A- 4 429 043
- US-A1- 2010 206 791

## Description

This invention relates to a method and to a device for the implementation of said method, to decompose and to selectively extract methane, carbon dioxide, NPK salts (nitrogen, phosphorus and potassium salts) of various titre and clarified water, from an organic matrix; said components will be the raw material for further industrial processes.

It is strongly felt the need to have systems for the reclamation of limited quantities of organic matrices, indicatively lower than 10 quintals per day (for example agro-food sub-products of manufacturing companies as well as small agro-zootechnical companies, large organized distribution (GDO), hotel, restaurant and catering (Ho.Re.Ca.) and residential settlements, suitable to supply continuous users (for example 20 kW for 24h, or even up to sizes of 1 kW, with for example only 50 kg per day). At the same time there is a general need for the supply of energy, better if by the simultaneous reclamation of organic materials, wastewaters, wet and grass cuttings arising from urban life. The costs and the inconveniences are relevant in the management of these reclamations, because of the specific treatments which must be subjected (along all the related passages), with poor containment of environmental and especially odour impacts. The environmental impact is usually not correctly assessed, as both CO₂ and CH₄ are odourless (natural evolutions of the organic material, already at ambient temperatures), but of great contribution to the greenhouse anthropic effect (especially the CH₄, produced in double size than CO₂ and 25 times more incisive than CO₂). The current state of the art includes anaerobic digesters normally directed to higher productions, with very high costs and generally ineffective for small size to which you think as potential applications of this invention. The digesters currently present on the market, moreover, does not offer effective solutions for the management of digestate and of other sub-products.

Other solutions such as the differentiated collection and the partial local treatments, are costly and ineffective on many fronts. They are not even detected solutions pointing at strategies of "distributed and pervasive solutions", to resolve the problems as close as possible to the source and in the respect of the general principle according to which "the polluter pays". These issues afflict many companies in the food and the agro-zootechnical sectors, that are in proximity to urban life. Document WO 2014075192 A1 has described a system and a method for producing biogas by decomposing organic matter, wherein the different steps of the process are carried out in a reactor having separating walls defining a sinuous path.

This invention aims at giving the best solution to all these issues. In particular it is not possible, by a single device, to produce multiple components directly usable, in particular with small volumes of organic matrices, because of the critical mass and of the actual technologies costs.

The purpose of this invention is to propose a method and a device for the implementation of said method, respectively conform to claims 1 and 5, to decompose an organic matrix and selectively extracting methane, carbon dioxide, NPK salts (nitrogen, phosphorus and potassium salt) of various titre and clarified water.

The method is characterized in that it includes the following steps:
- implementation of a hydrolytic phase, constituted by the fission action by means of the water, by hydration;
- implementation of a acidogenesis phase generated by means of specific bacteria;
- implementation of a acetogenesis phase generated by means of specific bacteria;
- implementation of a methanogenesis phase by means of specific bacteria, with a simultaneous gravimetric fission of mainly oleic phase, lighter and a predominantly protein phase, heavier;
- gravimetric separation of solutions of said NPK salts of different titres
- taking of clarified water.

The device is characterized in that it comprises a basin divided into various zones, in each of which biological reactions occur, in accordance with the described method, said zones being all communicating and identified by suitable separation baffles, in particular:
- a first baffle that extends from a first end of the basin to a second end of said basin, dividing it into two parts;
- a second baffle, of height equal to said first baffle that divides one of said parts in a first zone and a second zone extending from said first end of the basin until it reaches the vicinity of said second end of the basin, so that said two zones are communicating through an opening, of substantially vertical development, between the end of said second baffle and the second end of the basin;
- a plurality of baffles transversely arranged to said first baffle and inside a third zone, delimited by said first baffle, said third zone being placed in communication with said second zone through a transfer pipe, placed at about half height of said first baffle;
- two blocks, placed in the upper part of said basin and provided by taking means, each of said blocks comprising a plurality of vertical pipes and being fitted to carry out a gravimetric separation of the gases that are generated during the treatment of said mixture;
said baffles and said transfer pipe, by identifying a path crossed by the liquid mixture to be treated, that runs into the beginning of said first zone where it is placed an inlet duct of the liquid mixture to be treated and comes out from various points of said third zone.

Other characteristics, such as for example the possibility to improve the subtraction of hydrogen sulphides, by submersed homogeneous diffusers, by resorting to photosynthesis in absence of oxygen, will be the subject of the dependent claims.

The use of a device according to this invention allows, for example, to receive matrices of different concentrations and to optimize them by internal water, directly or by providing it to preliminary treatment, limiting the overall consumption of the primary water resource.

The invention finds application in the purification of water bodies and in the production of organic substances (protein fraction and oleic fraction) and NPK salts, said materials find use in the production of:
- biofuels;
- fertilizing mixtures for various industrial sectors (agro-zootechnical, chemical, etc.);
- hydrogen, as present in solution in the liquid phase;
- raw materials for vegetable crops (terrestrial or maritime);
- carbon dioxide for agronomic industries, metallurgical industries, fire services, agro-food and conservation.

The invention will now be described for illustrative and not limitative purpose, according to a preferred embodiment and with reference to the enclosed drawings, in which:
- the figure 1 is a perspective view of a device according to the invention;
- figure 2 shows, in two orthogonal views, the device according to the invention.

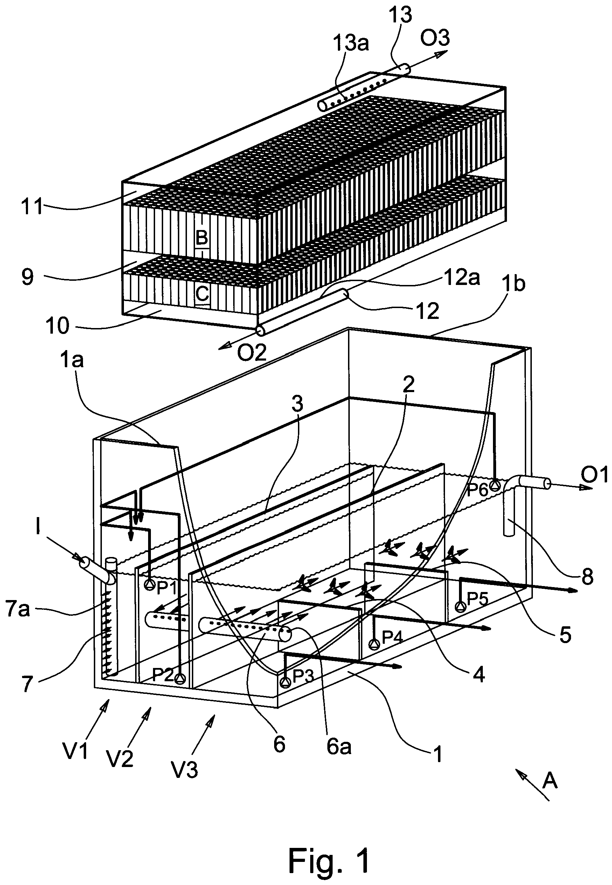

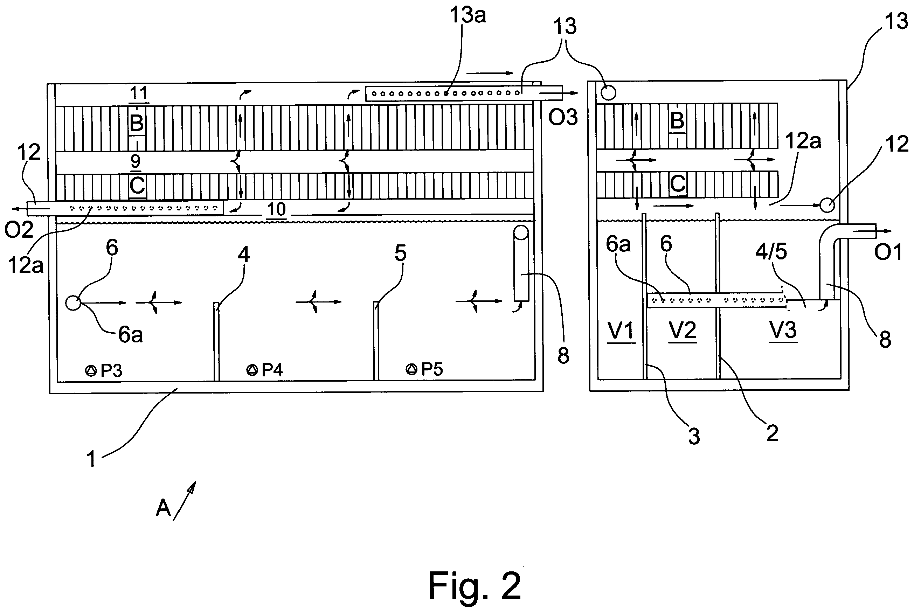

With reference to figures 1 and 2, with (A) it is indicated a device according to the invention, fitted to decompose and to separate the components of an organic matrix. Said device (A) includes a basin (1) divided into zones (V1), (V2) and (V3), in each of which biological reactions occur, said zones being all communicating and identified by suitable separation baffles. In particular:
- a first baffle (2) that extends from a first end (1a) of the basin (1) to a second end (1b) of said basin (1), subdividing it into two parts;
- a second baffle (3), having a height equal to said first baffle (2), that divides one of said parts in a first zone (V1) and in a second zone (V2), extending from said first end (1a) of the basin (1) until it reaches the vicinity of said second end (1b) of the basin (1), so that said two zones (V1) and (V2) are communicating through an opening, with a substantially vertical development, between the end of said second baffle (3) and the second end (1b) of the basin (1);
- a plurality of baffles (4), (5), having a height preferably equal to half of said first and second baffle (2), (3), transversely arranged to said first baffle (2) and inside a third zone (V3), delimited by said first baffle (2), said third zone (V3) being placed in communication with said second zone (V2) by means of a transfer pipe (6), positioned at about half the height of the first baffle (2), in which said transfer pipe (6) includes a tube closed at the ends and on which they have been made a plurality of holes (6a) aligned along a generatrix and facing towards the inside of the basin (1).

According to a preferred embodiment, said first baffle (2) divides the basin (1) into two substantially equal parts, while said second baffle (3) divides said first part, in said zones (V1) and (V2) in which the volume of the second zone (V2) is substantially double of the volume of the first zone (V1).

Said baffles (2) and (3) and said transfer pipe (6) identify a path crossed by the liquid mixture to be treated, that runs into the beginning of the first zone (V1), in which it is placed an inlet pipe (7) of the liquid mixture to be treated and comes out at various points of the third zone (V3).

At the bottom of the third zone (V3) it is positioned an outlet pipe (8), including a tube for the taking of clarified water to half the height of said first baffle (2).

Said first inlet pipe (7) includes a tube closed at the ends, with a Y shaped diversion, provided with a plurality of holes (7a), aligned along a generatrix and directed towards the corner of the basin (1), in order to favour the mixing and to avoid dead zones (or not operating). In addition, along said path, they are positioned some submerged pumps (P1,P2,P3,P4,P5,P6), whose function will be specified later on.

The basin (1), in the zone (V1) and (V2) is heated to a temperature suitable to favour the biological processes (typically comprised between 30 and 60°C). In order to sustain a slight excitation through convective motions, the basin (1) is thermally insulated on the outside and heated by heating means (not shown) positioned in the lower part of the basin itself.

In the upper part of the basin (1) they are placed two blocks (B) and (C), each of which includes a plurality of vertical pipes. The upper block (B), of greater development in height, is preferably made of plastic material (PE, PVC or similar) and the lower block (C), made of metal, are separated by a gap (9). The lower block (C) is cooled by means of a coil (not shown) crossed by a heat transfer fluid suitable to slightly lower the temperature of said lower block (C).

Said blocks (B) and (C) are inserted so as to obtain also a bottom gap (10) and an upper gap (11). At the ends of said gaps (10) and (11) they are positioned two pipes (12) and (13) respectively, at the antipodes and along the larger dimension of the basin (1), on which they are made a plurality of holes (12a) and (13a) respectively, aligned along a generatrix of said pipes.

To operate the described device (A) makes use of a hydraulic dynamic state of communicating vessels, so as to reduce the waste of mechanical energy for the necessary advancement movements of the fluid mixture.

The flow of the liquid mixture to be treated, indicated by the arrow (I), runs into the first zone (V1) of the basin (1), said mixture homogeneously spreading through the holes (7a) made on the first inlet tube (7). The mixture crosses through the various areas in which it is divided the basin (1), in which various reactions chemical-biological occur; in particular:
- in the first zone (V1) it is implemented the hydrolytic phase, constituted by the fission action of the water, by hydration, possibly also in high thermal conditions;
- in the final stretch of the first zone (V1) it is implemented the acidogenesis phase by specific bacteria;
- in the zone comprised between the first zone (V1) and the second zone (V2), it carries out a acetogenesis phase by specific bacteria;
- in the second area (V2) it is implemented a methanogenesis phase by specific bacteria (in this zone, because of the larger section, the flow speed will be lower in order to have a greater permanence time that favours the massive production of CH₄ and the gravimetric separation of one mainly oleic phase, lighter and a protein predominantly phase, heavier);
- In the end portion of the second zone (V2) it is taken a given quantity of liquid mixture through said submerged pumps (P1) and (P2) and it is recycled to the beginning of the zone (V1), in particular the pump (P1), placed in the upper part, recycles said mainly oleic mixture, lighter, while the pump (P2), placed in the lower part, recycles said protein predominantly mixture, heavier;
- in the transit from the second zone (V2) to the third zone (V3), through the pipe (6), placed at mid-height of the liquid phase, a laminar horizontal flow is obtained;
- in the third zone (V3), following the generation of said laminar horizontal flow, a widespread and different gravimetric action on the various components present in the mixture occurs, obtaining their deposition in the different sectors provided by means of the transversal baffles (4) and (5), from which they will be taken by said submerged pumps (P3), (P4) and (P5), said taking being constituted by NPK salts of different titres;
- in the terminal portion of the third zone (V3), after the deposition of the NPK salts, the liquid mass is composed almost exclusively of water, that is taken from said outlet pipe (8), said taking being carried out at about half the height of the liquid mass, in which the level of purity is higher;
- in the terminal portion of the third region (V3), at the top and in the proximity of the static surface of the liquid phase, it is taken the liquid still containing oleic traces, that is recycled at the beginning of the first zone (V1), said taking being obtained by said submerged pump (P6) and in suitable amount to the processes of dilution to be implemented at the beginning of the zone V1, to correctly condition the organic matrix.

The gases developed during the treatment (CH₄ and CO₂) collects in the gap (10) above the liquid mass and, from here, they are gravimetrically separate: the CO₂, heavier, tends to remain in the lower part, from which it will be extracted through the holes (12a) of the pipe (12), while the methane, lighter, will rise up to the upper gap (11), from which it will be extracted through the holes (13a) of the pipe (13).

Inside the gases developed by the reaction it is also present the steam which, passing through the vertical channels of the lower block (C), condensate and returns in liquid form in the fluid mass below, said condensation being obtained by the lowering of the temperature of said vertical channels of the lower block (C) obtained by said coil, as the lowering of the temperature allows to reach the saturation condition (dew point).

According to a preferred embodiment, in the final stretch of the first zone (V1) and in the zone between the first zone (V1) and the second zone (V2), in which the following steps are carried out the acidogenesis and the acetogenesis phases, a system of homogeneous and diffused lighting can find place, in order to prevent the formation of H₂S by a photosynthesis in absebce of oxygen.

## Claims

1. Method to decompose an organic matrix and selectively extracting methane, carbon dioxide, NPK salts (nitrogen, phosphorus and potassium salt) of various titre and clarified water, **characterized in that** it includes the following steps:
• implementation of a hydrolytic phase, constituted by the fission action by means of the water, by hydration;
• implementation of a acidogenesis phase generated by means of specific bacteria;
• implementation of a acetogenesis phase generated by means of specific bacteria;
• implementation of a methanogenesis phase by means of specific bacteria, with a simultaneous gravimetric separation of a mainly oleic phase, lighter and a predominantly protein phase, heavier;
• gravimetric separation of solutions of said NPK salts of different titres
• taking of clarified water.

2. Method to decompose an organic matrix, according to claim 1, **characterized by** the fact of providing for the taking of a mainly oleic mixture and of a mainly protein mixture at the end of said methanegenesis phase and to recycle said mixtures taken in the initial phase of the treatment of said organic matrix.

3. Method to decompose an organic matrix according to claim 1, **characterized by** the fact of providing for a horizontal laminar advancing fitted to facilitate said gravimetric separation of said NPK salts.

4. Method to decompose an organic matrix according to claim 1, **characterized by** the fact of providing for a taking of liquid still containing oleic traces, in the terminal phase of said treatment, said taken liquid being recycled at the beginning of said treatment.

5. Device (A) fitted to decompose an organic matrix and to selectively extract methane, carbon dioxide, NPK salts (salts of nitrogen, phosphorus and potassium) of various titre and clarified water, **characterized in that** it includes a basin (1) divided into zones (V1), (V2) and (V3), in each of which biological reactions occur, said zones being all communicating and located by suitable separation baffles. In particular:
• a first baffle (2) that extends from a first end (1a) of the basin (1) to a second end (1b) of said basin (1), subdividing it into two parts;
• a second baffle (3), having a height equal to said first baffle (2), that divides one of said parts in a first zone (V1) and in a second zone (V2), extending from said first end (1a) of the basin (1) until it reaches the vicinity of said second end (1b) of the basin (1), so that said two zones (V1) and (V2) are communicating through an opening, of a substantially vertical development, between the end of said second baffle (3) and the second end (1b) of the basin (1);
• a plurality of baffles (4), (5), transversely arranged to said first baffle (2) and inside a third zone (V3), delimited by said first baffle (2), said third zone (V3) being placed in communication with said second zone (V2) by means of a transfer pipe (6), positioned at about half the height of the first baffle (2), in which said transfer pipe (6) positioned at about half-height of aid first baffle (2);
• two blocks (B) and (C), positioned in the upper part of said basin (1) and provided with picking means (12, 12a, 13, 13a), each of said blocks (B) and (C) including a plurality of vertical pipes and being adapted to carry out a gravimetric separation of the gases that are generated during the treatment of said mixture;
said baffles (2) and (3) and said transfer pipes (6) defining a path crossed by the liquid mixture to be treated, which enters at the beginning of said first zone (V1), where it is placed an inlet duct (7) of the liquid mixture to be treated and comes out from various points of said third zone (V3).

6. Device (A) to decompose an organic matrix according to claim 5, **characterized in that** said first baffle (2) divides the basin (1) into two substantially equal parts.

7. Device (A) to decompose an organic matrix according to claim 5, **characterized in that** said second baffle (3) divides said first part, in said areas (V1) and (V2) in which the volume of the second zone (V2) is substantially double of the volume of the first zone (V1).

8. Device (A) to decompose an organic matrix according to claim 5, **characterized in that** said transfer pipe (6) includes a tube closed at the ends and on which they have been made a plurality of holes (6a) aligned along a generatrix and directed towards the inside of said basin (1).

9. Device (A) to decompose an organic matrix, according to claim 5, **characterized in that** said baffles (4), (5), arranged transversely to said first baffle (2) and inside said third zone (V3), are of a height substantially equal to half of said first and second baffle (2), (3).

10. Device (A) to decompose an organic matrix, according to claim 5, **characterized in that** said first inlet pipe (7) includes a tube closed at the ends, with a Y shaped diversion, provided with a plurality of holes (7a) aligned along a generatrix and directed towards the corner of the basin (1), in order to support the mixing and to avoid dead or not operating zones.

11. Device (A) to decompose an organic matrix, according to at least one of the claims from 5 to 10, **characterized in that** it provides heating means positioned in the lower part of said basin (1), in correspondence with the said zones (V1) and (V2), said basin (1) being thermally insulated and heated to a temperature suitable to sustain the biological processes (typically comprised between 30 and 60°C), with the purpose of supporting a slight excitation through convective motions.

12. Device (A) to decompose an organic matrix, according to at least one of the claims from 5 to 11, **characterized in that** said lower block (C) is made of metallic material, being provided means suitable to cool said lower block (C), so as to cause the condensation of the water vapour that passes through the vertical channels of said lower block (C), said water vapour developing from the fluid mass contained in said basin (1).

13. Device (A) to decompose an organic matrix, according to at least one of the claims from 5 to 12, **characterized in that** said taking means (12, 12a, 13, 13a) include pipes (12) and (13), on each of which a plurality of holes are made, (12a) and (13a) respectively, aligned along a generatrix of said pipes (12) and (13), said blocks (B) and (C) being positioned in such a way as to obtain an intermediate gap (9), a lower gap (10) and an upper gap (11) and said ducts (12) and (13) being positioned in said lower (10) and upper (11) gaps.

14. Device (A) to decompose an organic matrix, according to at least one of claims 5 to 13, **characterized by** the fact that:
• In said first zone (V1) of the basin (1) it is implemented a hydrolytic phase, constituted by the fission action by means of the water, by hydration, possibly also in high thermal conditions;
• in the final stretch of said first zone (V1) it is implemented the acidogenesis phase by means of specific bacteria;
• in a zone comprised between said first zone (V1) and said second zone (V2), it is carried out a acetogenesis phase, generated by means of specific bacteria;
• in said second zone (V2) it is implemented a methanogenesis phase by means of specific bacteria, with contemporaneous gravimetric separation of a mainly oleic phase, lighter and a protein predominantly phase, heavier;
• in a terminal stretch of the second zone (V2) it is taken a given quantity of liquid mixture through the submerged pumps (P1) and (P2) and it is recycled to the beginning of the zone (V1), in particular the pump (P1), placed in the upper part, recycles said mainly oleic mixture, lighter, while the pump (P2), placed in the lower part, recycles said protein predominantly mixture, heavier;
• in the transit from the second zone (V2) to the third zone (V3), through the pipe (6), placed at mid-height of the liquid phase, a laminar horizontal advancing is obtained;
• in said third zone (V3), following the generation of said laminar horizontal flow, a widespread and diffused gravimetric action, on the various components present in the mixture, occurs, obtaining their deposition in the different sectors provided by means of the transversal baffles (4) and (5), from which they will be taken by means of said submerged pumps (P3), (P4) and (P5), said taking being constituted by NPK salts of different titres;
• in a terminal stretch of the third zone (V3), after the deposition of the NPK salts, the liquid mass is composed almost exclusively of water, that is taken from said outlet pipe (8), said taking being carried out at about half the height of the liquid mass, in which the level of purity is higher;
• in said terminal stretch of said third region (V3), at the top and in the proximity of the static surface of the liquid phase, it is taken the liquid still containing oleic traces, that is recycled at the beginning of the first zone (V1), said taking being obtained by means of a submerged pump (P6) and in suitable amount to the processes of dilution to be implemented at the beginning of the first zone (V1), to correctly condition the organic matrix.

15. Device (A) to decompose an organic matrix, according to at least one of the claims from 5 to 14, **characterized by** the fact that in the final stretch of said first zone (V1) and in the zone included between said first zone (V1) and said second zone (V2), in which they are implemented said acidogenesis and acetogenesis phases it is placed a system of homogeneous and diffused lighting, in order to fight the formation of H₂S by photosynthesis in absence of oxygen.

## Patentansprüche

1. Methode für die Zerlegung einer organischen Matrix und die selektive Extraktion von Methan, Kohlendioxid, NPK-Salzen (Stickstoff-, Phosphor- und Kaliumsalze) verschiedener Art und geklärtem Wasser, die **dadurch gekennzeichnet ist, dass** sie folgende Phasen vorsieht:
• Ausführung einer Hydrolysephase, die aus dem Zerlegungsverfahren seitens des Wassers durch Hydratation besteht;
• Ausführung einer Versäuerungsphase, die seitens spezifischer Bakterien ausgeführt wird;
• Ausführung einer Essigbildenden Phase, die seitens spezifischer Bakterien ausgeführt wird;
• Ausführung einer Methanbildenden Phase seitens spezifischer Bakterien, mit kontextueller gravimetrischer Trennung einer vorwiegend ölhaltigen leichteren Phase und einer vorwiegend proteinhaltigen schwereren Phase;
• Gravimetrische Trennung von Lösungen der genannten NPK-Salze verschiedener Art
• Entnahme von geklärtem Wasser.

2. Methode für die Zerlegung einer organischen Matrix gemäß Patentanspruch 1, die **dadurch gekennzeichnet ist, dass** sie eine Entnahme einer vorwiegend ölhaltigen Mischung und einer vorwiegend proteinhaltigen Mischung am Ende der genannten Methanbildenden Phase und das Recycling der genannten Mischungen, die in der Anfangsphase der Behandlung der genannten organischen Matrix entnommen wurden, vorsieht.

3. Methode für die Zerlegung einer organischen Matrix gemäß Patentanspruch 1, die **dadurch gekennzeichnet ist, dass** sie einen horizontalen laminaren Vorschub auslöst, der dazu dient, die genannte gravimetrische Trennung der genannten NPK-Salze zu fordern.

4. Methode für die Zerlegung einer organischen Matrix gemäß Patentanspruch 1, die **dadurch gekennzeichnet ist, dass** sie eine Entnahme einer Flüssigkeit, die noch Ölspuren enthält, in der Endphase der genannten Behandlung vorsieht, die genannte entnommene Flüssigkeit wird am Anfang der genannten Behandlung recycelt.

5. Vorrichtung (A) für die Zerlegung einer organischen Matrix und die selektive Extraktion von Methan, Kohlendioxid, NPK-Salzen (Stickstoff-, Phosphor- und Kaliumsalze) verschiedener Art und geklärtem Wasser, die **dadurch gekennzeichnet ist, dass** sie einen Behälter (1) enthält, der in Zonen (V1), (V2) und (V3) aufgeteilt ist, in denen jeweils biologische Reaktionen erfolgen, die genannten Zonen sind alle verbunden und durch entsprechende Trennwände gekennzeichnet, insbesondere:
• Eine erste Wand (2), die sich von einem ersten Ende (1a) des Behälters (1) auf ein zweites Ende (1b) des genannten Behälters (1) ausdehnt und diesen in zwei Teile aufteilt;
• Eine zweite Wand (3) mit der gleichen Höhe wie die erste Wand (2), die einen der genannten Teile in eine erste Zone (V1) und eine zweite Zone (V2) aufteilt und sich vom ersten Ende (1a) des Behälters (1) ausdehnt, bis sie das zweite Ende (1b) des Behälters (1) erreicht, sodass die genannten zwei Zonen (V1) und (V2) miteinander durch einen Durchgang mit grundlegend vertikaler gestreckter Länge verbunden sind, der sich zwischen dem Ende der genannten zweiten Wand (3) und dem zweiten Ende (1b) des Behälters (1) befindet;
• Mehrere Wände (4), (5), die transversal zur genannten ersten Wand (2) und in der genannten dritten Zone (V3) angeordnet sind, welche durch die genannte erste Wand (2) abgegrenzt ist, die genannte dritte Zone (V3) wird mit der genannten zweiten Zone (V2) durch eine Übertragungsleitung (6) in Verbindung gebracht, die etwa in halber Höhe der genannten ersten Wand (2) angebracht ist;
• Zwei Blöcke (B) und (C), die im oberen Teil des genannten Behälters (1) angebracht und mit Entnahmevorrichtungen (12, 12a, 13, 13a) ausgestattet sind, jeder der genannten Blöcke (B) und (C) enthält mehrere vertikale Leitungen, die dazu dienen, eine gravimetrische Trennung der Gase auszuführen, die sich während der Behandlung der genannten Mischung entwickeln;
Die genannten Wände (2) und (3) und die genannte Übertragungsleitung (6) kennzeichnen einen Weg, der von der zu behandelnden flüssigen Mischung zurückgelegt wird, die am Anfang der genannten ersten Zone (V1) eintritt, in der die Eintrittsleitung (7) der zu behandelnden flüssigen Mischung angebracht ist, und die in verschiedenen Punkten der genannten dritten Zone (V3) austritt.

6. Vorrichtung (A) für die Zerlegung einer organischen Matrix gemäß Patentanspruch 5, die **dadurch gekennzeichnet ist, dass** die genannte erste Wand (2) den Behälter (1) in zwei grundlegend gleiche Teile aufteilt.

7. Vorrichtung (A) für die Zerlegung einer organischen Matrix gemäß Patentanspruch 5, die **dadurch gekennzeichnet ist, dass** die genannte zweite Wand (3) den genannten ersten Teil in die genannten Zonen (V1) und (V2) aufteilt, in denen das Volumen der zweiten Zone (V2) grundlegend doppelt so groß wie das Volumen der ersten Zone (V1) ist.

8. Vorrichtung (A) für die Zerlegung einer organischen Matrix gemäß Patentanspruch 5, die **dadurch gekennzeichnet ist, dass** die genannte Übertragungsleitung (6) eine an den Enden geschlossene Leitung enthält, an der mehrere Bohrungen (6a) angebracht wurden, die längs einer Mantellinie ausgerichtet und in Richtung des Inneren des genannten Behälters (1) gerichtet sind.

9. Vorrichtung (A) für die Zerlegung einer organischen Matrix gemäß Patentanspruch 5, die **dadurch gekennzeichnet ist, dass** die genannten Wände (4), (5), die transversal zur genannten ersten Wand (2) und in der genannten dritten Zone (V3) angeordnet sind, grundlegend halb so hoch wie die genannte erste und zweite Wand (2), (3) sind.

10. Vorrichtung (A) für die Zerlegung einer organischen Matrix gemäß Patentanspruch 5, die **dadurch gekennzeichnet ist, dass** die genannte erste Eintrittsleitung (7) eine an den Enden geschlossene Leitung mit Y-Zweigleitung enthält, die mit mehreren Bohrungen (7a) ausgestattet ist, die längs einer Mantellinie ausgerichtet und in Richtung der Ecke des Behälters (1) gerichtet sind, um die Mischung zu unterstützen und Totzonen oder inaktive Zonen zu vermeiden.

11. Vorrichtung (A) für die Zerlegung einer organischen Matrix gemäß mindestens einem der Patentansprüche von 5 bis 10, die **dadurch gekennzeichnet ist, dass** sie Heizvorrichtungen vorsieht, die im unteren Teil des genannten Behälters (1) an den genannten Zonen (V1) und (V2) angebracht sind, der genannte Behälter (1) ist isoliert und wird auf eine geeignete Temperatur erhitzt, um die biologischen Prozesse zu unterstützen (normalerweise zwischen 30 und 60 °C), mit dem Zweck, ein sanftes Schütteln durch konvektive Bewegungen zu unterstützen.

12. Vorrichtung (A) für die Zerlegung einer organischen Matrix gemäß mindestens einem der Patentansprüche von 5 bis 11, die **dadurch gekennzeichnet ist, dass** der genannte untere Block (C) aus Metallmaterial ist, es sind Vorrichtungen vorgesehen, die dazu dienen, den genannten unteren Block (C) zu kühlen, um die Kondensation des Wasserdampfes auszulösen, der durch die vertikalen Leitungen des genannten unteren Blocks (C) strömt, der genannte Wasserdampf entwickelt sich durch die flüssige Masse, die im genannten Behälter (1) enthalten ist.

13. Vorrichtung (A) für die Zerlegung einer organischen Matrix gemäß mindestens einem der Patentansprüche von 5 bis 12, die **dadurch gekennzeichnet ist, dass** die genannten Entnahmevorrichtungen (12, 12a, 13, 13a) Leitungen (12) und (13) enthalten, an denen jeweils mehrere Bohrungen, jeweils (12a) und (13a), angebracht sind, die längs einer Mantellinie der genannten Leitungen (12) und (13) ausgerichtet sind, die genannten Blöcke (B) und (C) sind so positioniert, dass sie einen Zwischenhohlraum (9), einen unteren Hohlraum (10) und einen oberen Hohlraum (11) bilden und dass die genannten Leitungen (12) und (13) in dem genannten unteren (10) und in dem genannten oberen Hohlraum (11) positioniert sind.

14. Vorrichtung (A) für die Zerlegung einer organischen Matrix gemäß mindestens einem der Patentansprüche von 5 bis 13, die **dadurch gekennzeichnet ist, dass**:
• in der genannten ersten Zone (V1) des Behälters (1) eine Hydrolysephàse ausgeführt wird, die aus dem Trennungsverfahren seitens des Wassers durch Hydratation, ggf. auch bei hohen Temperaturbedingungen, erfolgt;
• im genannten Endabschnitt der genannten ersten Zone (V1) eine Versäuerungsphase erfolgt, die durch spezifische Bakterien ausgeführt wird;
• in einer Zone zwischen der genannten ersten Zone (V1) und der genannten zweiten Zone (V2) eine Essigbildende Phase erfolgt, die durch spezifische Bakterien ausgeführt wird;
• in der genannten zweiten Zone (V2) eine Methanbildende Phase seitens spezifischer Bakterien, mit kontextueller gravimetrischer Trennung einer vorwiegend ölhaltigen leichteren Phase und einer vorwiegend proteinhaltigen schwereren Phase, ausgeführt wird;
• in einem Endabschnitt der genannten zweiten Zone (V2) eine gegebene Menge der flüssigen Mischung durch Tauchpumpen (P1) und (P2) entnommen wird, die am Anfang der genannten ersten Zone (V1) recycelt wird, insbesondere die Pumpe (P1), die im oberen Teil angebracht ist, recycelt die genannte leichtere vorwiegend ölhaltige Mischung, während die Pumpe (P2), die im unteren Teil angebracht ist, die genannte schwerere vorwiegend proteinhaltige Mischung recycelt;
• im Durchgang von der genannten zweiten Zone (V2) zur genannten dritten Zone (V2) durch die Leitung (6), die in halber Höhe der flüssigen Phase angebracht ist, ein horizontaler laminarer Vorschub ausgelöst wird;
• in der genannten dritten Zone (V3) infolge der Auslösung der genannten horizontalen laminaren Bewegung eine diffuse und unterschiedliche gravimetrische Wirkung auf die verschiedenen Komponenten erfolgt, die in der Mischung vorhanden sind, welche ihre Ablagerung in den verschiedenen Abteilungen durch die transversalen Wände (4) und (5) hervorruft, aus denen sie durch die genannten Tauchpumpen (P3), (P4) und (P5) entnommen werden, die genannte Entnahme besteht aus NPK-Salzen verschiedener Art;
• in einem Endabschnitt der genannten dritten Zone (V3) nach der Ablagerung der NPK-Salze die flüssige Masse fast ausschließlich aus Wasser besteht, das aus der genannten Austrittsleitung (8) entnommen wird, die genannte Entnahme wird in etwa halber Höhe der flüssigen Masse ausgeführt, in der der Reinheitsgrad höher ist;
• im genannten Endabschnitt der genannten dritten Zone (V3) oben und am statischen Spiegel der flüssigen Phase Flüssigkeit entnommen wird, die noch Ölspuren enthält, die am Anfang der ersten Zone (V1) recycelt wird, die genannte Entnahme erfolgt durch eine Tauchpumpe (P6) in geeigneter Menge zu den Verdünnungsprozessen, die am Anfang der ersten Zone (V1) ausgeführt werden, um die organische Matrix korrekt zu beeinflussen.

15. Vorrichtung (A) für die Zerlegung einer organischen Matrix gemäß mindestens einem der Patentansprüche von 5 bis 14, die **dadurch gekennzeichnet ist, dass** im Endabschnitt der genannten ersten Zone (V1) und in der Zone zwischen der genannten ersten Zone (V1) und der genannten zweiten Zone (V2), in der die genannten Versäuerungsphasen und Essigbildenden Phasen ausgeführt werden, ein homogenes und diffuses Beleuchtungssystem angebracht ist, das dazu dient, der H2S-Bildung entgegenzuwirken, die durch anoxygene Fotosynthese erfolgt.

## Revendications

1. Méthode pour décomposer une matrice organique et extraire sélectivement du méthane, du gaz carbonique, des sels NPK (sels d'azote, de phosphore et de potasse) de différents titres et de l'eau clarifiée, **caractérisée en ce qu'**il prévoit les phases suivantes :
• réalisation d'une phase hydrolytique composée par l'action de scission par l'eau, à travers l'hydratation ;
• réalisation d'une phase acide-génique produite par des bactéries spécifiques ;
• réalisation d'une phase acétogénique produite par des bactéries spécifiques ;
• réalisation d'une phase méthanogénique produite par des bactéries spécifiques, avec la séparation gravimétrique contextuelle d'une phase essentiellement oléique, plus légère, et d'une phase essentiellement protéique, plus lourde ;
• séparation gravimétrique de solution des sels NPK avec différents titres ;
• prélèvement d'eau clarifiée.

2. Méthode pour décomposer une matrice organique, selon la revendication 1, **caractérisée en ce qu'**elle prévoit un prélèvement d'un mélange essentiellement oléique et d'un mélange essentiellement protéique à l'issue d'une phase méthanogénique et de recycler ces mélanges prélevés en phase initiale du traitement de la matrice organique.

3. Méthode pour décomposer une matrice organique, selon la revendication 1, **caractérisée en ce qu'**elle détermine un avancement laminaire horizontal apte à favoriser la séparation gravimétrique des sels NPK.

4. Méthode pour décomposer une matrice organique, selon la revendication 1, **caractérisée en ce qu'**elle prévoit un prélèvement de liquide contenant encore des traces oléiques, en phase terminale du traitement ; le liquide prélevé est recyclé au début du traitement.

5. Dispositif (A) pour décomposer une matrice organique et extraire sélectivement du méthane, du gaz carbonique, des sels NPK (sels d'azote, de phosphore et de potasse) de différents titres et de l'eau clarifiée, **caractérisé en ce qu'**il comprend une cuve (1) partagée en zones (V1), (V2) et (V3), dans chacune desquelles se vérifient des réactions biologiques ; ces zones sont communicantes et séparées par des cloisons, en particulier :
• une première cloison (2) qui s'étend d'une première extrémité (1a) de la cuve (1) à une seconde extrémité (1b) de la cuve (1), en la séparant en deux parties ;
• une deuxième cloison (3), de même hauteur que la première cloison (2), qui sépare l'une des parties en une première zone (V1) et une seconde zone (V2), en s'étendant de la première extrémité (1a) de la cuve (1) jusqu'à atteindre la seconde extrémité (1b) de la cuve (1), de sorte que les deux zones (V1) et (V2) soient communicantes à travers un passage, se développant substantiellement en vertical, compris entre l'extrémité de la seconde cloison (3) et la seconde extrémité (1b) de la cuve (1);
• une pluralité de cloisons (4), (5), placées transversalement par rapport à la première cloison (2) et situées à l'intérieur d'une troisième zone (V3), délimitée par la première cloison (2) ; la troisième zone (V3) est mise en communication avec la seconde zone (V2) au moyen d'un tube de transfert (6) placé environ à mi-hauteur de la première cloison (2) ;
• deux blocs (B) et (C), situés dans la partie supérieure de la cuve (1) qui sont pourvus de moyens de prélèvement (12, 12a, 13, 13a) ; chaque bloc (B) et (C) comprend une pluralité de tubes verticaux aptes à effectuer une séparation gravimétrique des gaz qui se développent pendant le traitement du mélange ;
deux cloisons (2) et (3) et le tube de transfert (6) déterminent le parcours effectué par le mélange liquide qui entre au début de la première zone (V1), où se trouve le tube d'entrée (7) du mélange liquide à traiter puis sort à différents endroits de la troisième zone (V3).

6. Dispositif (A) pour décomposer une matrice organique, selon la revendication 5, **caractérisé en ce que** la première cloison (2) sépare la cuve (1) en deux parties substantiellement égales.

7. Dispositif (A) pour décomposer une matrice organique, selon la revendication 5, **caractérisé en ce que** la seconde cloison (3) sépare la première partie, en zones (V1) et (V2) où le volume de la seconde zone (V2) est substantiellement le double du volume de la première zone (V1).

8. Dispositif (A) pour décomposer une matrice organique, selon la revendication 5, **caractérisé en ce que** le tube de transfert (6) comprend un tube fermé aux extrémités qui est doté d'une pluralité de trous (6a) alignés le long d'une ligne génératrice et qui sont tournés vers l'intérieur de la cuve (1).

9. Dispositif (A) pour décomposer une matrice organique, selon la revendication 5, **caractérisé en ce que** les cloisons (4), (5), disposées transversalement par rapport à la première cloison (2) et à l'intérieur de la troisième zone (V3), ont une hauteur substantiellement égale à la moitié de la hauteur de la première (2) et de la deuxième cloison (3).

10. Dispositif (A) pour décomposer une matrice organique, selon la revendication 5, **caractérisé en ce que** le premier tube d'entrée (7) comprend un tube fermé aux extrémités, avec dérivation en Y, doté d'une pluralité de trous (7a), alignés le long d'une ligne génératrice et tournés vers l'angle de la cuve (1), afin de soutenir le mélange et d'éviter les zones mortes ou inactives.

11. Dispositif (A) pour décomposer une matrice organique, selon l'une des revendications de 5 à 10, **caractérisée en ce qu'**elle prévoit des moyens chauffants placés dans la partie inférieure de la cuve (1), au niveau des zones (V1) et (V2) ; la cuve (1) est calorifugée et chauffée à une température adéquate pour soutenir les processus biologiques (typiquement comprise entre 30 et 60°C), afin de soutenir une légère agitation à travers des mouvements convectifs.

12. Dispositif (A) pour décomposer une matrice organique, selon l'une des revendications de 5 à 11, **caractérisé en ce que** le bloc inférieur (C) est en métal ; des moyens aptes à refroidir le bloc inférieur (C) ont été prévus de sorte à provoquer la condensation de la vapeur d'eau qui passe à travers les canaux verticaux du bloc inférieur (C) ; ainsi la vapeur d'eau se développe à partir de la masse fluide contenue dans la cuve (1).

13. Dispositif (A) pour décomposer une matrice organique, selon l'une des revendications de 5 à 12, **caractérisé en ce que** les moyens de prélèvement (12, 12a, 13, 13a) comprennent des tubes (12) et (13) ; chaque tube est doté, d'une pluralité de trous, (12a) et (13a) respectivement, alignés le long d'une ligne génératrice des tubes (12) et (13) ; les blocs (B) et (C) sont positionnés de sorte à obtenir un interstice intermédiaire (9), un interstice inférieur (10) et un interstice supérieur (11); les tubes (12) et (13) sont positionnés dans l'interstice inférieur (10) et supérieur (11).

14. Dispositif (A) pour décomposer une matrice organique, selon l'une des revendications de 5 à 13, **caractérisé en ce que** :
• dans la première zone (V1) de la cuve (1), se produit une phase hydrolytique, constituée par l'action de scission par l'eau, à travers l'hydratation, voire en conditions thermiques élevées ;
• au bout de la première zone (V1) se produit une phase acidogénique produite par des bactéries spécifiques ;
• dans une zone comprise entre la première zone (V1) et la seconde zone (V2), se produit une phase acétogénique, produite par des bactéries spécifiques ;
• dans la seconde zone (V2) se produit une phase méthanogénique produite par des bactéries spécifiques, avec la séparation gravimétrique contextuelle d'une phase essentiellement oléique, plus légère, et d'une phase essentiellement protéique, plus lourde ;
• au bout de la seconde zone (V2) une quantité donnée de mélange liquide est prélevée par des pompes submergées (P1) et (P2) puis elle est recyclée au début de la première zone (V1) ; la pompe (P1), située dans la partie supérieure, recycle le mélange essentiellement oléique, plus léger tandis que la pompe (P2), placé dans la partie inférieure, recycle le mélange essentiellement protéique, plus lourd ;
• lors du passage de la seconde zone (V2) à la troisième zone (V3), à travers le tube (6), situé à mi-hauteur de la phase liquide, un avancement laminaire horizontal se produit ;
• dans la troisième zone (V3), après la vérification du mouvement laminaire horizontal, se produit une action diffuse gravimétrique sur les différents composants présents dans le mélange, en entraînant leur dépôt dans les différents secteurs prédisposés à l'aide des cloisons transversales (4) et (5) d'où ils seront prélevés par les pompes submergées (P3), P4) et (P5) ; ce prélèvement est constitué de sels NPK de différents titres ;
• au bout de la troisième zone (V3), après le dépôt des sels NPK, la masse liquide est composée presque exclusivement d'eau qui est prélevée à travers le tube de sortie (8) ; ce prélèvement est exécutée à mi-hauteur de la masse liquide où le niveau de pureté est plus élevé ;
• au bout de la troisième zone (V3), en haut et à proximité du niveau statique de la phase liquide, on prélève le liquide contenant encore des traces oléiques qui est recyclé au début de la première zone (V1) : le prélèvement est effectué par une pompe submergée (P6) en quantité appropriée aux processus de dilution à réaliser au début de la première zone (V1), pour conditionner correctement la matrice organique.

15. Dispositif (A) pour décomposer une matrice organique, selon l'une des revendications de 5 à 14, **caractérisé en ce qu'**au bout de la première zone (V1) et dans le morceau compris entre la première zone (V1) et la seconde zone (V2), où se produisent les phases acidogéniques et acétogéniques, se trouve un système d'éclairage homogène et diffus, apte à contrer la formation de H₂S par photosynthèse anoxygénique.
